# EUROPEAN PATENT APPLICATION

(11) **EP 3 206 026 A1**
(43) Date of publication of application: **16.08.2017**
(21) Application number: 17154948.8
(22) Date of filing: 07.02.2017
(51) Int. Cl.: G01N 31/22, G01N 33/00

(54) **METHOD AND ARRANGEMENT FOR DETECTING SULPHIDE GENERATING IN A STRUCTURE**

(30) Priority: 10.02.2016 FI 20164021 U
(71) Applicant: Sisäilmatutkimuspalvelut Elisa Aattela Oy, 33100 Tampere (FI)
(72) Inventor: Aattela, Elisa, 33100 Tampere (FI)
(74) Representative: Kolster Oy Ab

(57) **Abstract**

In the solution put forth, an indication piece is adapted in a cavity in a structure. The indication piece is such that it reacts to inorganic sulphide so that if the indication piece is exposed to inorganic sulphide, a detectable change is formed on its outer surface. The indication piece may be of such a material such as copper, for example, that its surface is darkened when the indication piece is exposed to inorganic sulphide. The indication piece is left in the cavity in the structure for a specific time. When the indication piece has been in the cavity for the desired time, an organoleptic examination, for example, may be carried out as to whether a detectable change has been formed on the outer surface of the indication piece.

## Description

### Background of the invention

The invention relates to a method and arrangement for detecting sulphide generating in a structure.

Inorganic sulphides include, for example, hydrogen sulphide and carbon disulphide. Hydrogen sulphide, for example, is in large concentrations as much as life-threatening. Even in relatively low concentrations, hydrogen sulphide causes detrimental health effects, such as nausea, dizziness, breathing difficulties, cough, sore throat and/or headache. Likewise, carbon disulphide causes detrimental health effects.

Inorganic sulphide may be created when, for example, a structure that includes gypsum is moist and there are microbes that degrade gypsum, that is, bacteria that degrade gypsum, in connection with the structure. Structures that include gypsum are, for example, gypsum boards, sprayed gypsum, filler that includes gypsum, and concrete from which moisture cannot escape.

If inorganic sulphide is generated from the structures of a building, people staying in such a building are subjected to its detrimental health effects. Therefore, the need exists for a simple arrangement for detecting inorganic sulphide generating in a structure.

### Brief description of the invention

The purpose of this invention is to bring forth a new type of method and arrangement for detecting sulphide generating in a structure.

The solution according to the invention is characterised by what is disclosed in the independent claims. Some embodiments of the invention are disclosed in the dependent claims.

In the solution put forth, an indication piece is adapted into a cavity in the structure. The indication piece is such that it reacts to inorganic sulphide so that as the piece is exposed to inorganic sulphide, a detectable change takes place on its outer surface. The indication piece may be, for example, of a material such as copper that its surface is darkened when the indication piece is exposed to inorganic sulphide. The indication piece is left in the cavity in the structure for a particular time period, which may vary from a few days to a few months, for example. When the indication piece has been in the cavity for the desired time, an organoleptic examination may be carried out as to whether a detectable change has appeared on the outer surface of the indication piece. If no detectable change has taken place after a sufficiently long time, the conclusion can be made that at least in the vicinity of the spot in question no harmful amount of inorganic sulphide is generated from the structure. If, instead, there is a detectable change on the outer surface of the indication piece, the indication piece may be sent to be examined. Measurement may then confirm whether the change in the outer surface was caused by the effects of sulphide, and this way it may be made sure whether the structure is such that inorganic sulphide is generated in it. By such a simple a reliable way, it may be detected whether hydrogen sulphide or carbon disulphide, for example, are generated in the structure. Further, it is possible to pinpoint the place in the building, where inorganic sulphide is generated.

### Brief description of the drawings

The invention will now be described in closer detail in connection with the preferred embodiments, with reference to the accompanying drawings, in which:
Figure 1 shows a schematic cross-sectional side view of an arrangement for detecting inorganic sulphide,
Figure 2 shows a schematic cross-sectional side view of another arrangement for detecting inorganic sulphide, and
Figure 3 shows schematically a solution according to which the generation of inorganic sulphide in a structure is detected.

### Detailed description of the invention

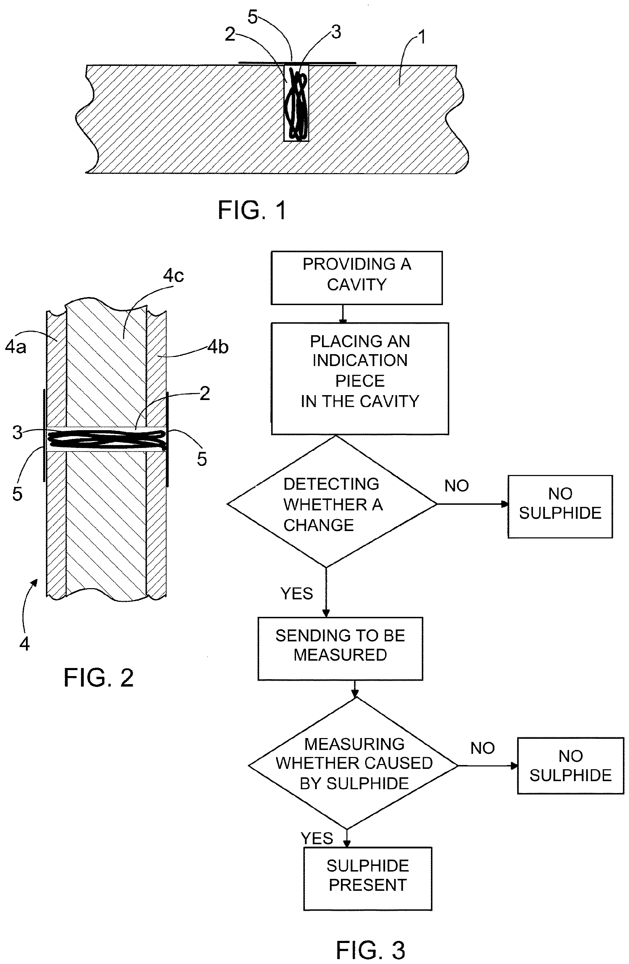

Figure 1 shows schematically a part of a structure of a building. In the embodiment of Figure 1, the structure in question is a concrete slab 1. The concrete slab 1 may be the base floor or intermediate floor of a building, for example.

The concrete slab 1 has a cavity 2. The cavity 2 may be a cavity drilled in the concrete slab 1. The cavity 2 may be an existing cavity in the structure. Such a cavity may be, for example, a shrinkage gap in a construction joint.

The diameter of the cavity 2 may be 6 to 20 mm, for example. The length of the cavity may be 2 to 15 cm, for example.

Into the cavity 2, an indication piece 3 has been placed. Before the indication piece 3 is installed, the cavity 2 is cleaned. In particular, if the cavity 2 is formed by drilling, for example, the cavity 2 must be cleaned. The cavity 2 may be cleaned by vacuuming, for example.

Once the indication piece 3 has been placed in the cavity 2, the cavity 2 is closed or covered. When the cavity 2 is closed, the indication piece 3 is more efficiently subjected to the effects of the substances emitted from the structure than if the cavity 2 were left open. The means used to close the cavity 2 may be duct tape 5, for example.

The indication piece 3 is such that is reacts to inorganic sulphide so that as the piece 3 is exposed to inorganic sulphide, a detectable change takes place on its outer surface. The indication piece may be, for example, of such a material that its surface is darkened when the indication piece 3 is exposed to inorganic sulphide.

The indication piece 3 may be, for example, of copper whose surface becomes darker in a clearly detectable manner when it is exposed to inorganic sulphide. Further, the indication piece 3 may be of another suitable material on whose outer surface a clearly detectable change takes place when it is exposed to inorganic sulphide. Such materials include silver and silver-plated copper, for example.

The indication piece 3 may be formed of a folded wire, for example. The diameter of the wire may be, for example, 0.5 to 2 mm. The indication piece 3 may also be formed of a strip or folded strip.

In Figure 2, a wall 4 is presented as the structure of a building, by way of example. The wall 4 of Figure 2 has a first gypsum board 4a, a second gypsum board 4b, and, between the gypsum boards 4a and 4b, an intermediate structure 4c known per se, shown in Figure 2 only schematically for reasons of clarity.

The cavity 2 is a hole drilled through the wall 4. After this, the cavity 2 has been cleaned. The cavity 2 is closed at both ends by duct tape 5.

In the embodiment of Figure 2, what may happen is that when the indication piece 3 has been in the cavity 4 for the desired time, a detectable change has taken place on its outer surface only on the part of the indication piece 3 in the vicinity of the second gypsum board 4b, for example. If the appeared change is confirmed by a measurement to have been caused by the effect of inorganic sulphide, it is in this case possible to very precisely determine that the location in the structure where inorganic sulphide is generated is the second gypsum board 4b. This way, the generation of inorganic sulphide from the structure may be pinpointed very accurately.

The solution of how the generation of inorganic sulphide in a structure is detected will in the following be described with reference to the diagram in Figure 3. The inorganic sulphide to be detected may be, for example, hydrogen sulphide H₂S and/or sulphocarbide, that is, carbon disulphide CS₂. Inorganic sulphide may be generated when gypsum structures caught in an oxygen-free space, moistened, and including calcium sulphate are degraded into inorganic sulphides, such as hydrogen sulphide and carbon disulphide, by bacteria that simplify sulphates.

At the first phase, the structure is provided with a cavity. Providing a cavity may mean that a cavity existing in the structure is selected to be used for detecting sulphide, or a new cavity is formed in the structure by drilling, for example.

After the cavity is provided, it may be cleaned. The cleaning of the cavity may take place by vacuuming, for example.

Next, an indication piece is placed in the cavity. After that, if desired, the cavity may be closed or covered.

The indication piece is left in the cavity for a particular time period so that it is possible to determine whether a detectable change caused by sulphide takes place on the indication piece. This time period may vary from a few days to a few months, for example.

When the indication piece has been in the cavity for the desired time, an organoleptic examination, for example, is carried out as to whether a detectable change has been formed on the outer surface of the indication piece. If it is evident that no visible change, for example, has taken place on the outer surface of the indication piece, the conclusion can be made, if so desired, that in the spot in question no sulphide is generated in the structure.

If, instead, an organoleptic change has taken place, the indication piece is sent for examination by measuring to a laboratory, for example. If desired, such an organoleptic examination phase may be skipped and every indication piece that has spent a required time in the cavity be sent to be examined by measuring.

After this, the indication piece is examined by measuring. Such an examination may take place by an EDS device, that is, energy-dispersive x-ray spectroscopy equipment, connected to an electron microscope. So, such a measurement is used to examine whether a change on the outer surface of the indication piece is caused by inorganic sulphide. If the measurements indicate that the change was not caused by sulphide, it may again be noted that at the location in question no sulphide is generated in the structure. If, on the other hand, the measurement result indicates that the change is caused by sulphide, it may be concluded that at the location in question, sulphide is generated in the structure.

For persons skilled in the art on the one hand, and for individual consumers on the other hand, a product and service entity described below may be compiled. At first, an indication piece is so provided that it is reliable as regards its characteristics for detecting sulphide. Such an indication piece may be delivered in an appropriately sealed enclosure, whereby the reliability of detecting is ensured. Operating procedures may be delivered in connection with the indication piece. The operating instructions may include, for example, instructions for providing, cleaning and closing the cavity, instructions for the appropriate detection period, and instructions for sending the indication piece for examination. Further, a reply-paid envelope may be sent with the indication piece, in which the indication piece placed in said enclosure, for example, may be delivered for examination.

It is obvious for a person skilled in the art that as technology advances the basic idea of the invention may be implemented in a plurality of ways. The invention and its embodiments are therefore not restricted to the examples described in the above but may vary within the scope of the claims.

## Claims

1. An arrangement for detecting sulphide generating in a structure, the arrangement including a cavity in the structure and an indication piece adaptable in the cavity, the indication piece being such that it reacts to sulphide so that if the indication piece is exposed to sulphide, a detectable change appears on its outer surface.

2. An arrangement as claimed in claim 1, wherein the indication piece is of such a material that its surface is darkened if the indication piece is exposed to sulphide.

3. An arrangement as claimed in claim 1 or 2, wherein the cavity is a cavity drilled in the structure.

4. An arrangement as claimed in any one of the previous claims, wherein the indication piece consist of a folded wire.

5. An arrangement as claimed in any one of the previous claims, wherein the indication piece is of silver.

6. An arrangement as claimed in any one of the previous claims, which further includes means for closing the cavity.

7. A method for detecting sulphide generating in a structure, the method having the steps of providing the structure with a cavity, placing into the cavity an indication piece which is such that it reacts to sulphide so that if the indication piece is exposed to sulphide, a detectable change appears on its outer surface, leaving the indication piece in the cavity for a specific time period, and, after the time period in question, examining whether a change has taken place on the outer surface of the indication piece.

8. A method as claimed in claim 7, in which method the indication piece is examined by measuring to find out whether the change is caused by sulphide.

9. A method as claimed in claim 7, in which method an organoleptic examination takes place as to whether a change has appeared on the outer surface of the indication piece.

10. A method as claimed in claim 9, in which method if it is detected by an organoleptic examination that a change has taken place on the outer surface of the indication piece, the indication piece is examined by measuring to find out whether the change is caused by sulphide.

11. A method as claimed in any one of the previous claims 7 to 10, in which method, after the providing of the cavity, it is cleaned before the indication piece is installed.

12. A method as claimed in any one of the previous claims 7 to 11, in which method the cavity is covered after the placing of the indication piece.
